# EUROPEAN PATENT APPLICATION

(11) **EP 4 163 269 A1**
(43) Date of publication of application: **12.04.2023**
(21) Application number: 21816971.2
(22) Date of filing: 31.05.2021
(51) Int. Cl.: C07C 51/44, C07C 57/04

(54) **METHOD FOR PRODUCING EASILY POLYMERIZABLE COMPOUND**

(30) Priority: 05.06.2020 JP 2020098799
(71) Applicant: Nippon Shokubai Co., Ltd., Osaka-shi, Osaka 541-0043 (JP)
(72) Inventor: KASE, Yuki, Himeji-shi, Hyogo 671-1282 (JP); SUGIMOTO, Takashi, Himeji-shi, Hyogo 671-1282 (JP)
(74) Representative: Müller-Boré & Partner Patentanwälte PartG mbB
(86) International application number: PCT/JP2021/020693
(87) International publication number: WO 2021/246368

(57) **Abstract**

A method for producing an easily polymerizable compound, comprising a polymerization prevention step of supplying an oxygen-containing gas to an easily polymerizable compound-containing liquid through a gas supply means having one or more supply ports, wherein an area S (mm²) per one supply port is 0.1 or more and 20 or less, and a ratio V/S of a linear velocity V (m/s) of the gas at the supply port to the area S in the polymerization preventing step is 6.0 or more.

## Description

### TECHNICAL FIELD

The present invention relates to a method for producing an easily polymerizable compound such as (meth)acrylic acid or (meth)acrylic acid ester.

### BACKGROUND ART

Conventionally, a method of preventing polymerization of an easily polymerizable compound by supplying an oxygen-containing gas to an easily polymerizable compound such as (meth)acrylic acid or (meth)acrylic acid ester is known. For example, Patent Literature 1 discloses a method of supplying an oxygen-containing gas to an acrylic acid-containing liquid to be supplied to a distillation column or at a bottom part of a distillation column when purifying acrylic acid by distillation. Patent Literature 2 discloses a method of supplying an oxygen-containing gas to a (meth)acrylic acid-containing liquid or a (meth)acrylic acid ester-containing liquid in a pipe when transferring (meth)acrylic acid or (meth)acrylic acid ester to a tank after purification by distillation.

### CITATION LIST

### PATENT LITERATURE

PATENT LITERATURE 1
   Japanese Unexamined Patent Application Publication No. 2003-277318
PATENT LITERATURE 2
   Japanese Unexamined Patent Application Publication No. 2016-028108

### SUMMARY OF INVENTION

### TECHNICAL PROBLEM

When preventing polymerization by supplying an oxygen-containing gas to an easily polymerizable compound-containing liquid, it is required that the oxygen-containing gas is reliably supplied to the easily polymerizable compound-containing liquid. For example, supplying the oxygen-containing gas to the easily polymerizable compound-containing liquid is carried out using a gas supply means such as a nozzle, and it is desired that a gas supply port of the gas supply means is not clogged, or if clogging occurs, it can be resolved without stopping operation.

The present invention has been made in view of the above circumstances, and an object of the present invention is to provide a method for producing an easily polymerizable compound while preventing polymerization of the easily polymerizable compound by supplying an oxygen-containing gas, that can effectively suppress or eliminate clogging of a supply port of a gas supply means for supplying the oxygen-containing gas.

### SOLUTION TO PROBLEM

The present invention includes the following inventions.
[1] A method for producing an easily polymerizable compound, comprising a polymerization prevention step of supplying an oxygen-containing gas to an easily polymerizable compound-containing liquid through a gas supply means having one or more supply ports, wherein
   an area S (mm²) per one supply port is 0.1 or more and 20 or less, and
   a ratio V/S of a linear velocity V (m/s) of the gas at the supply port to the area S in the polymerization preventing step is 6.0 or more.
[2] The method for producing an easily polymerizable compound according [1], further comprising a cleaning step of supplying a gas to the gas supply means to clean the supply port, wherein
   a pressure difference across the supply port of the gas supply means in the polymerization prevention step is 100 kPa or lower, and
   a pressure difference across the supply port of the gas supply means in the cleaning step is 150 kPa or higher.
[3] The method for producing an easily polymerizable compound according to [1] or [2], further comprising a cleaning step of supplying a liquid to the gas supply means to clean the supply port.
[4] A method for producing an easily polymerizable compound, comprising a polymerization prevention step of supplying an oxygen-containing gas to an easily polymerizable compound-containing liquid through a gas supply means having one or more supply ports, and a cleaning step of supplying a gas to the gas supply means to clean the supply port, wherein
   a pressure difference across the supply port of the gas supply means in the polymerization prevention step is 100 kPa or lower, and
   a pressure difference across the supply port of the gas supply means in the cleaning step is 150 kPa or higher.
[5] A method for producing an easily polymerizable compound, comprising a polymerization prevention step of supplying an oxygen-containing gas to an easily polymerizable compound-containing liquid through a gas supply means having one or more supply ports, and a cleaning step of supplying a liquid to the gas supply means to clean the supply port.
[6] The method for producing an easily polymerizable compound according to any one of [1] to [5], wherein the polymerization prevention step is conducted in at least one of the following manners (1) to (4):
   (1) the polymerization prevention step is conducted in a step of introducing an easily polymerizable compound-containing gas into a cooling column selected from an absorption column and a condensation column to obtain the easily polymerizable compound-containing liquid, and
      the gas supply means is provided in the cooling column or a circulation line for withdrawing a part of the easily polymerizable compound-containing liquid from the cooling column and returning it to the cooling column;
   (2) the polymerization prevention step is conducted in a step of introducing the easily polymerizable compound-containing liquid to a vaporization separation column selected from a distillation column and a stripping column to purify, and
      the gas supply means is provided in the vaporization separation column, a supply line for supplying the easily polymerizable compound-containing liquid to the vaporization separation column, or a circulation line for withdrawing a part of the easily polymerizable compound-containing liquid from the vaporization separation column and returning it to the vaporization separation column;
   (3) the polymerization prevention step is conducted in a step of storing the easily polymerizable compound-containing liquid in a storage tank, and
      the gas supply means is provided in the storage tank, a supply line for supplying the easily polymerizable compound-containing liquid to the storage tank, or a circulation line for withdrawing a part of the easily polymerizable compound-containing liquid from the storage tank and returning it to the storage tank;
   (4) the polymerization prevention step is conducted in a step of introducing the easily polymerizable compound-containing liquid in a reactor and reacting it, and
      the gas supply means is provided in the reactor, a supply line for supplying the easily polymerizable compound-containing liquid to the reactor, or a circulation line for withdrawing a part of the easily polymerizable compound-containing liquid from the reactor and returning it to the reactor.

### ADVANTAGEOUS EFFECTS OF INVENTION

According to the present invention, in producing an easily polymerizable compound while preventing polymerization of the easily polymerizable compound by supplying an oxygen-containing gas, clogging of a supply port of a gas supply means for supplying the oxygen-containing gas can be effectively suppressed or eliminated.

### DESCRIPTION OF THE PREFERRED EMBODIMENTS

A method for producing an easily polymerizable compound of the present invention comprises a polymerization prevention step of supplying an oxygen-containing gas to an easily polymerizable compound-containing liquid. For an easily polymerizable compound, it is important to prevent an unintended polymerization reaction during its production or handling, and as a method for preventing polymerization of an easily polymerizable compound, a method of supplying an oxygen-containing gas to an easily polymerizable compound-containing liquid is known. Meanwhile, supplying the oxygen-containing gas to the easily polymerizable compound-containing liquid is carried out using a gas supply means such as a nozzle, and it is desired that a supply port of gas provided with the gas supply means is not narrowed or completely blocked. For example, if the easily polymerizable compound-containing liquid enters the inside of the gas supply means from the supply port, the easily polymerizable compound-containing liquid comes into contact with a supply flow of the oxygen-containing gas, and the easily polymerizable compound-containing liquid evaporates to solidify, whereby deposits are likely to be generated and accumulated inside the gas supply means or at the supply port. As a result, the size of the supply port of the gas supply means may be narrowed, or the supply port may be completely blocked, resulting in insufficient supply of the oxygen-containing gas to the easily polymerizable compound-containing liquid. In the method for producing an easily polymerizable compound of the present invention, when supplying the oxygen-containing gas to the easily polymerizable compound-containing liquid, clogging of the supply port of the gas supply means or generation or accumulation of deposits can be suppressed.

The easily polymerizable compound is not particularly limited as long as it is a compound of which polymerization reaction easily proceeds in a liquid, and examples thereof include (meth)acrylic acid and its ester, maleic anhydride and its ester, acrylonitrile, styrene, divinyl benzene, vinyl toluene, diethylene glycol monovinyl ether, and others. Among them, typical easily polymerizable compounds include (meth)acrylic acid and its esters. Examples of (meth)acrylic acid ester include, for example, methyl (meth)acrylate, ethyl (meth)acrylate, butyl (meth)acrylate, isobutyl (meth)acrylate, t-butyl (meth)acrylate, 2-ethylhexyl (meth)acrylate, nonyl (meth)acrylate, 2-hydroxyethyl (meth)acrylate, 2-hydroxypropyl (meth)acrylate, ethylene glycol di(meth)acrylate, polyethylene glycol di(meth)acrylate, trimethylolpropane di(meth)acrylate, trimethylolpropane tri(meth)acrylate, 2-(2-vinyloxyethoxy)ethyl (meth)acrylate, methoxyethyl (meth)acrylate, benzyl (meth)acrylate, glycidyl (meth)acrylate, and others. Preferable examples of the easily polymerizable compound include (meth)acrylic acid, butyl (meth)acrylate, isobutyl (meth)acrylate, t-butyl (meth)acrylate, 2-ethylhexyl (meth)acrylate, 2-hydroxyethyl (meth)acrylate, 2-hydroxypropyl (meth)acrylate, methoxyethyl (meth)acrylate, nonyl (meth)acrylate, ethylene glycol di(meth)acrylate, polyethylene glycol di(meth)acrylate, trimethylolpropane di(meth)acrylate, trimethylolpropane tri(meth)acrylate, 2-(2-vinyloxyethoxy)ethyl (meth)acrylate, benzyl (meth)acrylate and glycidyl (meth)acrylate.

It is preferable that the easily polymerizable compound-containing liquid is a liquid obtained in a process for producing an easily polymerizable compound and contains an easily polymerizable compound. For example, in the case where the easily polymerizable compound is (meth)acrylic acid, examples of the easily polymerizable compound-containing liquid include a crude (meth)acrylic acid-containing liquid recovered as a liquid by collection or condensation of (meth)acrylic acid-containing gas obtained by catalytic gas-phase oxidation reaction of a (meth)acrylic acid production raw material, a purified (meth)acrylic acid-containing liquid obtained by purifying the crude (meth)acrylic acid-containing liquid, a process liquid obtained during purification of the crude (meth)acrylic acid-containing liquid, a temporarily storage liquid of the crude (meth)acrylic acid-containing liquid or the purified (meth)acrylic acid-containing liquid, and others, but is not limited to the above.

A concentration of an easily polymerizable compound in the easily polymerizable compound-containing liquid is not particularly limited, and may be, for example, 10 mass% or more, 20 mass% or more, 30 mass% or more, or 40 mass% or more. From the viewpoint of necessity of preventing polymerization of the easily polymerizable compound by supplying the oxygen-containing gas, the concentration of an easily polymerizable compound in the easily polymerizable compound-containing liquid is preferably 50 mass% or more, more preferably 60 mass% or more, even more preferably 70 mass% or more, and still even more preferably 80 mass% or more. The upper limit of the concentration of an easily polymerizable compound in the easily polymerizable compound-containing liquid is not particularly limited, and it may be, for example, 99.9 mass% or less, 99 mass% or less, or 97 mass% or less.

In the polymerization prevention step, an oxygen-containing gas is supplied to the easily polymerizable compound-containing liquid through a gas supply means having one or more supply ports. The oxygen-containing gas is supplied to the easily polymerizable compound-containing liquid through the supply port provided in the gas supply means.

The oxygen-containing gas is not particularly limited as long as it contains molecular oxygen, and pure oxygen gas, air, a gas obtained by mixing nitrogen, oxygen and air, having any oxygen concentration, or the like can be used. A concentration of molecular oxygen in the oxygen-containing gas may be arbitrarily adjusted, for example, in the range of 1 volume% to 100 volume%.

Examples of the gas supply means include a nozzle, a diffusion tube, a diffusion plate, and the like, and the shape thereof is not particularly limited. The gas supply means is installed by being immersed in the easily polymerizable compound-containing liquid. The supply port is arranged facing the outside of the gas supply means, and is provided so as to be in contact with the easily polymerizable compound-containing liquid outside the gas supply means. The supply port is provided as an opening through which the oxygen-containing gas is supplied into the easily polymerizable compound-containing liquid.

The supply port of the gas supply means may be provided so as to face upward, downward, or sideways. In the case where the gas supply means comprises a gas supply pipe and the supply port is provided in the gas supply pipe, an extending direction of the gas supply pipe at the location where the supply port is provided is not particularly limited, and the gas supply pipe at that may be installed so as to extend upward, downward, laterally, or diagonally upward or downward. The gas supply pipe may be installed as a straight pipe, or may have one or more bends, taking into consideration the structure of a location of the gas supply pipe. The extending direction of the gas supply pipe (for example, whether the pipe extending vertically is upward or downward) is determined based on the direction in which the gas flows in the gas supply pipe.

For example, in the case where the gas supply pipe is installed so as to extend upward and the supply port is provided so as to face upward, even if the easily polymerizable compound-containing liquid flows back into the gas supply pipe, the back-flowing liquid can be discharged from a lower part of the gas supply pile, and liquid accumulation in the gas supply pipe can be suppressed. In addition, it is possible to prevent deposits with low specific gravity from flowing into the gas supply pipe.

In the case where the gas supply pipe is installed so as to extend upward and the supply port is provided so as to face sideways, even if the easily polymerizable compound-containing liquid flows back into the gas supply pipe, the back-flowing liquid can be discharged from the lower part of the gas supply pipe, and accumulation of the easily polymerizable compound-containing liquid (hereinafter may be simply referred to as "liquid accumulation") in the gas supply pipe can be suppressed. In addition, it is possible to prevent deposits with high specific gravity from flowing into the gas supply pipe.

In the case where the gas supply pipe is installed so as to extend downward and the supply port is provided so as to face downward, even if the easily polymerizable compound-containing liquid flows back into the gas supply pipe, the back-flowing liquid can be discharged from the lower part, namely, the supply port, of the gas supply pipe, and liquid accumulation in the gas supply pipe can be suppressed.

In the case where the gas supply pipe is installed so as to extend downward and the supply port is provided so as to face sideways, even if the easily polymerizable compound-containing liquid flows back into the gas supply pipe, the back-flowing liquid can be discharged from the lower part, namely, the supply port, of the gas supply pipe, and liquid accumulation in the gas supply pipe can be suppressed. In addition, it is possible to prevent deposits with high specific gravity from flowing into the gas supply pipe.

In the case where the gas supply pipe is installed so as to extend laterally, since a plurality of supply ports can be provided at substantially the same height, there is an advantage that liquid pressure around each supply port (that is, pressure of the easily polymerizable compound-containing liquid by liquid depth) is equal. In addition to that, in the case where the supply port is provided so as to face upward, it is possible to prevent deposits with low specific gravity from flowing into the gas supply pipe. Meanwhile, in the case where the supply port is provided so as to face downward, even if the easily polymerizable compound-containing liquid flows back into the gas supply pipe, the back-flowing liquid can be discharged from the supply port, and liquid accumulation in the gas supply pipe can be suppressed. In the case where the supply port is provided so as to face sideways, it is possible to prevent deposits with high specific gravity from flowing into the gas supply pipe.

In the case where the gas supply pipe is installed so as to extend diagonally downward and the supply port is provided so as to face upward, it is possible to prevent deposits with low specific gravity from flowing into the gas supply pipe.

In the case where the gas supply pipe is installed so as to extend diagonally downward and the supply port is provided so as to face downward, even if the easily polymerizable compound-containing liquid flows back into the gas supply pipe, the back-flowing liquid can be discharged from the lower part, namely, the supply port, of the gas supply pipe, and liquid accumulation in the gas supply pipe can be suppressed.

In the case where the gas supply pipe is installed so as to extend diagonally downward and the supply port is provided so as to face sideways, it is possible to prevent deposits with high specific gravity from flowing into the gas supply pipe.

The supply port provided in the gas supply means preferable has an area S (mm²) per one supply port of 0.1 or more and 20 or less. By setting the area of the supply port to 0.1 mm² or larger, the supply port comes to be formed in a somewhat large size, and clogging of the supply port is less likely to occur. Meanwhile, by setting the area of the supply port to 20 mm² or smaller, coupled with the relationship with a linear velocity V of a gas at the supply port described later, it is less likely occur that a portion of the easily polymerizable compound-containing liquid flows backward and enters the inside of the gas supply means through the supply port, during the oxygen-containing gas is supplied through the supply port. As a result, it is possible to suppress clogging of the supply port or the inside of the gas supply means due to formation of a polymer or solidification of the easily polymerizable compound or a minor component contained in the easily polymerizable compound-containing liquid. In addition, a bubble diameter of the oxygen-containing gas supplied from the supply port into the easily polymerizable compound-containing liquid does not become too large, and the oxygen-containing gas easily dissolves in the easily polymerizable compound-containing liquid. The area of the supply port is more preferably 0.3 mm² or larger, even more preferably 0.5 mm² or larger, and more preferably 18 mm² or smaller, even more preferably 15 mm² or smaller.

The oxygen-containing gas is preferably supplied from the supply port to the easily polymerizable compound-containing liquid so that a ratio V/S of a linear velocity V (m/s) of the oxygen-containing gas at the supply port to the area S (mm²) per one supply port is 6.0 or more. That is, it is preferable to set the linear velocity V of the oxygen-containing gas at the supply port to increase as the area S of the supply port increases so that the ratio V/S is 6.0 or more. By setting the ratio V/S in this manner, it is less likely occur that a portion of the easily polymerizable compound-containing liquid flows backward and enters the inside of the gas supply means through the supply port, during the oxygen-containing gas is supplied through the supply port. As a result, formation of deposits inside the gas supply means or at the supply port and clogging of the supply port are less likely to occur. The ratio V/S is more preferably 6.2 or more, and even more preferably 6.4 or more. The upper limit of the ratio V/S is not particularly limited, and may be, for example, 100 or less, 60 or less, 40 or less, or 30 or less.

The shape of the supply port is not particularly limited, however, from the viewpoint that the easily polymerizable compound-containing liquid is less likely to enter the inside of the gas supply means through the supply port during the oxygen-containing gas is supplied through the supply port, shapes without corners are preferable, and for example, circular shape or elliptical shape is preferable. Further, by forming the supply port in such a shape, formation of deposits at the supply port is less likely to occur.

It is preferable that a pressure difference across the supply port of the gas supply means in the polymerization prevention step is 100 kPa or lower. Thereby, the specifications of a blower or a compressor for supplying the oxygen-containing gas can be suppressed, and facility costs and utility costs for the blower or the compressor can be reduced. The pressure difference across the supply port of the gas supply means in the polymerization prevention step is more preferably 50 kPa or lower, even more preferably 20 kPa or lower, still even more preferably 10 kPa or lower, and particularly preferably 5 kPa or lower. The lower limit of the pressure difference is not particularly limited as long as the ratio V/S described above is 6.0 or more, and may be, for example, 0.01 kPa or higher, 0.05 kPa or higher, or 0.1 kPa or higher.

It is preferable that the gas supply means is connected to a gas supply line, and the oxygen-containing gas is supplied to the gas supply means through the gas supply line. The gas supply line is preferably provided with a flow meter and a valve, whereby the supply amount of the oxygen-containing gas from the gas supply means can be adjusted. The gas supply line may be branched into a plurality of branches, and each branched gas supply line may be provided with the gas supply means. In this case, it is preferable that each of the branched supply lines is provided with a flow meter and a valve so that the amount of the oxygen-containing gas supplied from each gas supply means can be adjusted. By adjusting the oxygen-containing gas to be supplied from each gas supply means in a predetermined amount or more, clogging of the supply port of each gas supply means can be prevented. When the supply amount of oxygen-containing gas from some gas supply means decreases, it can be judged that deposits are generated inside the gas supply means or at the supply port, and by increasing opening degree of the valve of the supply line connected to that gas supply means, it is possible to increase the supply amount of the oxygen-containing gas and prevent the supply port of the gas supply means from being clogged.

From the viewpoint of preventing backflow from the supply port to the inside of the gas supply means, a surface tension of the easily polymerizable compound-containing liquid at 20°C is preferably 10 mN/m or more, more preferably 15 mN/m or more, and even more preferably 20 mN/m or more. The upper limit of the surface tension is not particularly limited, and may be, for example, 200 mN/m or less, 150 mN/m or less, or 100 mN/m or less. For example, the surface tension of acrylic acid at 20°C is 28.1 mN/m.

The easily polymerizable compound-containing liquid preferably contains a polymerization inhibitor. Thereby, the effect of preventing polymerization in the easily polymerizable compound-containing liquid is enhanced, in combination with the supply of the oxygen-containing gas to the easily polymerizable compound-containing liquid. As the polymerization inhibitor, conventionally known polymerization inhibitors can be used, and examples of the polymerization inhibitor includes, for example, quinone compounds such as hydroquinone and methoquinone (p-methoxyphenol); phenothiazine compounds such as phenothiazine, bis-(α-methylbenzyl)phenothiazine, 3,7-dioctylphenothiazine and bis-(α-dimethylbenzyl)phenothiazine; N-oxyl compounds such as 2,2,6,6-tetramethylpiperidinooxyl, 4-hydroxy-2,2,6,6-tetramethylpiperidinooxyl and 4,4',4"-tris-(2,2,6,6-tetramethylpiperidinooxyl)phosphite; copper salt compounds such as copper dialkyl dithiocarbamate, copper acetate, copper naphthenate, copper acrylate, copper sulfate, copper nitrate and copper chloride; manganese salt compounds such manganese dialkyl dithiocarbamate, manganese diphenyl dithiocarbamate, manganese formate, manganese acetate, manganese octanoate and manganese naphtenate; nitroso compounds such as N-nitrosophenyl hydroxylamine and its salts, p-nitrosophenol, and N-nitrosodiphenylamine and its salts; and the like. These polymerization inhibitors may be used alone or in combination of two or more. Among them, it is preferable to use at least quinones as the polymerization inhibitor.

Polymerization inhibitor concentration in the easily polymerizable compound-containing liquid may be appropriately set, and is preferably 1 mass ppm or more, and more preferable 10 mass ppm or more, even more preferably 100 mass ppm or more, still even more preferably 1000 mass ppm or more, and preferably 50000 mass ppm or less, more preferably 10000 mass ppm or less, even more preferably 5000 mass ppm or less, still even more preferably 2000 ppm mass or less.

The easily polymerizable compound-containing liquid may contain other compounds such as dimers of an easily polymerizable compound, maleic acid, glyoxal, acetic acid, formaldehyde, acrolein, propionic acid, furfural, benzaldehyde and derivatives thereof, as long as the amount of other compounds is small.

It is preferable that the method for producing an easily polymerizable compound of the present invention further comprises a cleaning step of the gas supply means, in addition to the polymerization prevention step of supplying the oxygen-containing gas to the easily polymerizable compound-containing liquid through the gas supply means. When the oxygen-containing gas is continuously supplied to the easily polymerizable compound-containing liquid through the gas supply means for a long period of time (for example, several weeks) in the polymerization prevention step, deposits gradually accumulate at the supply port of the gas supply means, and there is a risk of narrowing the size of the supply port or complete blockage of the supply port. Therefore, it is preferable to provide a cleaning step of the gas supply means after supplying the oxygen-containing gas or between supplying of the oxygen-containing gas to remove deposits adhered to the inside of the gas supply means or the supply port.

In the cleaning step, it is preferable that a gas is supplied to the gas supply means to clean the supply port. Hereinafter, the gas supplied in the cleaning step is referred to as a "cleaning gas". The cleaning gas is supplied from the inside of the gas supply means toward the outside of that, and at this time, the cleaning gas is supplied at a pressure higher than a supply pressure of the oxygen-containing gas in the polymerization prevention step. For example, a pressure difference across the supply port of the gas supply means in the cleaning step is preferably 150 kPa or higher, more preferably 160 kPa or higher, and even more preferably 180 kPa or higher. By supplying the cleaning gas at such a pressure, deposits adhered to the inside of the gas supply means or the supply port can be efficiently removed. Meanwhile, the upper limit of the pressure difference of that is not particularly limited; however, If the supply pressure of the cleaning gas is too high, a high-specification compressor may be required or the facility may need to be pressure-resistant, and therefore, the pressure difference of that is preferably 1000 kPa or lower, more preferable 800 kPa or lower. The pressure difference across the supply port means the pressure difference between the inside and the outside of the gas supply means.

The kind of the cleaning gas is not particularly limited, and it is preferable to use a non-condensable gas. As the cleaning gas, for example, the above-described oxygen-containing gas can be used, or an inert gas such as nitrogen gas may be used.

It is preferable that the cleaning gas is supplied in a short period of time. For example, a duration of one supply of the cleaning gas is preferably 20 seconds or shorter, more preferably 10 seconds or shorter, and even more preferably 5 seconds or shorter. Furthermore, it is preferable to supply the cleaning gas multiple times in such a short period of time. By supplying the cleaning gas in this manner, deposits adhered to the supply port of the gas supply means can be efficiently removed. The duration of one supply of the cleaning gas may be 3 seconds or shorter, 2 seconds or shorter, or 1 second or shorter.

A linear velocity of the cleaning gas at the supply port of the gas supply means may be appropriately set according to the pressure difference across the supply port, and preferably, for example, 400 m/s or more, more preferably 450 m/s or more, and even more preferably 500 m/s or more at maximum. The upper limit of the linear velocity is not particularly limited, and may be, for example, 2000 m/s or less, 1500 m/s or less, or 1200 m/s or less.

In the cleaning step, it is also preferable that a liquid is supplied to the gas supply means to clean the supply port. By supplying a liquid from the inside of the gas supply means toward the outside of that, deposits adhered to the inside of the gas supply means or the supply port can be removed. As the liquid, an easily polymerizable compound-containing liquid is preferably used, and specifically, an easily polymerizable compound-containing liquid obtained in an easily polymerizable compound production process in which the gas supply means is installed is preferably used. By using the easily polymerizable compound-containing liquid as the cleaning medium, quality fluctuation of the easily polymerizable compound-containing liquid in which the gas supply means is installed can be suppressed.

As the easily polymerizable compound-containing liquid used as the cleaning medium, an easily polymerizable compound-containing liquid whose main component has the same composition as that of the easily polymerizable compound-containing liquid flowing near the supply port is preferable. For example, an easily polymerizable compound-containing liquid supplied to an apparatus provided with the gas supply means, an easily polymerizable compound-containing liquid flowing near the supply port, an easily polymerizable compound-containing liquid discharged from an apparatus provided with the gas supply means, and the like are mentioned. The easily polymerizable compound-containing liquid used as the cleaning medium may be an easily polymerizable compound-containing liquid obtained from different locations in the same production line, including a product, or may be an easily polymerizable compound-containing liquid obtained from different production lines if there are a plurality of production lines.

In the case of using a liquid as the cleaning medium in the cleaning step, a linear velocity of the liquid at the supply port of the gas supply means is preferably 0.01 m/s or more, more preferably 0.05 m/s or more, and even more preferably 0.1 m/s or more. Thereby, deposits adhered to the inside of the gas supply means or the supply port can be efficiently removed. Meanwhile, the upper limit of the linear velocity of that is preferably 10 m/s or less, more preferably 5 m/s or less, and even more preferably 1 m/s or less, whereby adverse effects on the easily polymerizable compound-containing liquid in which the gas supply means is installed (e.g., obstruction of the flow of the easily polymerizable compound-containing liquid) can be suppressed.

The polymerization prevention step and the cleaning step described above can be conducted in any step in the process for producing an easily polymerizable compound. Hereinafter, examples of specific embodiments of the present invention are described with a case of a process for producing (meth)acrylic acid.

In the case that the easily polymerizable compound is (meth)acrylic acid, the method for producing an easily polymerizable compound of the present invention (in this case, the method for producing (meth)acrylic acid) preferably comprises, for example, the steps of: obtaining a (meth)acrylic acid-containing gas by subjecting a (meth)acrylic acid production raw material to a catalytic gas phase oxidation reaction; and introducing the (meth)acrylic acid-containing gas into a cooling column selected from an absorption column and a condensation column to obtain a (meth)acrylic acid-containing liquid.

The (meth)acrylic acid production raw material to be subjected to the catalytic gas-phase oxidation reaction can be used without any particular limitation as long as (meth)acrylic acid is formed by reaction, and examples thereof include propane, propylene, (meth)acrolein, isobutylene and others. Acrylic acid can be obtained by, for example, oxidizing propane, propylene or acrolein in one step, or oxidizing propane or propylene via acrolein in two steps. Acrolein is not limited to that obtained by oxidizing propane or propylene, which is used as a raw material, and acrolein may be obtained by dehydrating glycerin of a raw material, for example. Methacrylic acid can be obtained by, for example, oxidizing isobutylene or methacrolein in one step, or oxidizing isobutylene via methacrolein in two steps.

As a catalyst used for the catalytic gas phase oxidation, a conventionally known catalyst can be used. For example, in the case where propylene is used as a raw material for producing acrylic acid, a complex oxide catalyst containing molybdenum and bismuth (molybdenum-bismuth catalyst) is preferably used as the catalyst. In the case where propane or acrolein is used as a raw material for producing acrylic acid, a complex oxide catalyst containing molybdenum and vanadium (molybdenum-vanadium catalyst) is preferably used as the catalyst.

As a reactor for performing the catalytic gas phase oxidation reaction, a fixed bed reactor, a fluidized bed reactor, a moving bed reactor, or the like can be used. Among these, from the viewpoint of excellent reaction efficiency, a multitubular fixed bed reactor is preferably used. In the case where (meth)acrylic acid is produced by oxidizing a (meth)acrylic acid production raw material in two steps, a reactor for performing a first oxidation reaction and a rector for performing a second oxidation reaction are combined or one reactor is divided into a region where the first oxidation reaction is performed and a region where the second oxidation reaction is performed, whereby (meth)acrylic acid is produced from a (meth)acrylic acid production raw material. In the latter case, for example in a fixed bed reactor, a catalyst for performing the first oxidation reaction may be filled in an inlet side (namely, an introducing side of the (meth)acrylic acid production raw material) of reaction tubes of the fixed bed reactor, and a catalyst for performing the second oxidation reaction may be filled in an outlet side of those.

The reaction for producing the (meth)acrylic acid-containing gas from the (meth)acrylic acid production raw material may be conducted under known reaction conditions. For example, in the case of producing acrylic acid from propylene by oxidation reaction in two steps, a propylene-containing gas is introduced into a reactor together with molecular oxygen, and for example, the first oxidation reaction may be performed under conditions of reaction temperature of 250°C to 450°C, reaction pressure of 0 MPaG to 0.5 MPaG and space velocity of 300 h⁻¹ to 5000 h⁻¹, and then the second oxidation reaction may be performed under conditions of reaction temperature of 250°C to 380°C, reaction pressure of 0 MPaG to 0.5 MPaG, and space velocity of 300 h⁻¹ to 5000 h-1.

The (meth)acrylic acid-containing gas obtained by catalytic gas-phase oxidation reaction of the (meth)acrylic acid production raw material is introduced into an absorption column or a condensation column, thereby obtaining a (meth)acrylic acid-containing liquid. In the former case, as the (meth)acrylic acid-containing gas is introduced into an absorption column and brought into contact with a collection solvent, (meth)acrylic acid is absorbed into the collection solvent, thereby obtaining the (meth)acrylic acid-containing liquid (collection step). In the latter case, as the (meth)acrylic acid-containing gas is introduced into a condensation column and cooled, (meth)acrylic acid is condensed, thereby obtaining the (meth)acrylic acid-containing liquid (condensation step). In the present specification, an absorption column and a condensation column may be collectively called a "cooling column".

The absorption column is not particularly limited as long as the (meth)acrylic acid-containing gas can be brought into contact with the collection solvent in the absorption column. For example, as the (meth)acrylic acid-containing gas is introduced into the absorption column from a lower part of the absorption column and the collection solvent is introduced into the absorption column from an upper part of the absorption column, the (meth)acrylic acid-containing gas comes into countercurrent contact with the collection solvent while rising in the absorption column, and (meth)acrylic acid is absorbed by the collection solvent and recovered as the (meth)acrylic acid-containing liquid. As the absorption column, for example, a tray column provided with shelves (sieve trays) in the column, a packed column filled with a packing in the column, a wet wall column in which the collection solvent is supplied to the surface of the inner wall of the column, a spray column in which the collection solvent is sprayed into the space in the column or the like can be adopted.

The collection solvent is not particularly limited as long as it can absorb and dissolve (meth)acrylic acid, and for example, diphenyl ether, diphenyl, a mixture of diphenyl ether and diphenyl, water, (meth)acrylic acid-containing water (for example, an aqueous solution containing (meth)acrylic acid obtained in the production process of (meth)acrylic acid), or the like can be used. Among them, water or (meth)acrylic acid-containing water is preferably used as the collection solvent, and more preferably, water or (meth)acrylic acid-containing water containing 50 mass% or more (more preferably 70 mass% or more, even more preferably 80 mass% or more) of water.

Temperature and the feed amount of the collection solvent may be appropriately set so that (meth)acrylic acid contained in the (meth)acrylic acid-containing gas is sufficiently absorbed by the collection solvent. Temperature of the collection solvent is preferably 0°C or higher, more preferably 5°C or higher, and preferably 35°C or lower, more preferably 30°C or lower, from the viewpoint of increasing collection efficiency of (meth)acrylic acid. Regarding the feed amount of the collection solvent, a liquid gas ratio (L/G) indicated by the ratio of the feed amount (L) of the collection solvent to the absorption column with respect to the introducing amount (G) of the (meth)acrylic acid-containing gas to the absorption column is preferably 2 L/m³ or more, more preferably 3 L/m³ or more, even more preferably 5 L/m³ or more, and preferably 15 L/m³ or less, more preferably 12 L/m³ or less, even more preferably 10 L/m³ or less.

(Meth)acrylic acid absorbed by the collection solvent is withdrawn from the absorption column as the (meth)acrylic acid-containing liquid. The (meth)acrylic acid-containing liquid may be withdrawn from, for example, a position below a supply position of the (meth)acrylic acid-containing gas in the absorption column (for example, the bottom of the absorption column).

It is preferred that the absorption column is provided with a circulation line for returning a part of the (meth)acrylic acid-containing liquid discharged from the absorption column to the absorption column. The circulation line is preferably provided so as to connect a position below a supply position of the (meth)acrylic acid-containing gas in the absorption column and a positon above the supply position of the (meth)acrylic acid-containing gas and a discharge position of the (meth)acrylic acid-containing liquid and below a supply position of the collection solvent in the absorption column. By returning a part of the (meth)acrylic acid-containing liquid withdrawn from the absorption column to the absorption column through the circulation line and circulating it, (meth)acrylic acid concentration in the (meth)acrylic acid-containing liquid can be increased. The circulation line is preferably provided with a heat exchanger for cooling the (meth)acrylic acid-containing liquid flowing through the circulation line.

Meanwhile, in the case where the (meth)acrylic acid-containing liquid is obtained by condensing the (meth)acrylic acid-containing gas, a condensation column is preferably used. As the condensation column, one equipped with a heat exchanger having a heat transfer surface can be used, for example. By cooling the (meth)acrylic acid-containing gas via the heat transfer surface, (meth)acrylic acid can be condensed from the (meth)acrylic acid-containing gas, and as a result, the (meth)acrylic acid-containing liquid is obtained. A conventionally known heat exchanger may be used as the heat exchanger, and for example, a plate heat exchanger, a multitubular (shell-and-tube) heat exchanger, a double-tube heat exchanger, a coil heat exchanger, a spiral plate heat exchanger, or the like can be employed. A plurality of heat exchangers may be connected in series and the (meth)acrylic acid-containing gas may be cooled in multiple stage to perform fractional condensation, thereby recovering the (meth)acrylic acid-containing liquid.

The condensation column may be configured such that the (meth)acrylic acid-containing liquid is obtained from the (meth)acrylic acid-containing gas by bringing the (meth)acrylic acid-containing gas into contact with a condensate. In this case, it is preferable to provide a shelves (sieve trays) in the condensation column or fill the condensation column with a packing to improve contact efficiency between the (meth)acrylic acid-containing gas and the condensate. By using such a condensation column, the (meth)acrylic acid-containing gas is separated and condensed as the (meth)acrylic acid-containing gas is introduced into the condensation column from a lower part of the condensation column and moves from the lower part to the upper part of the condensation column, for example. At this time, the (meth)acrylic acid-containing liquid is withdrawn from a middle stage of the condensation column, substances having a higher boiling point than (meth)acrylic acid is withdrawn from a lower stage of the condensation column and substances having a lower boiling point than (meth)acrylic acid is withdrawn from an upper stage of the condensation column, for example.

The condensation column may be provided with a circulation line for withdrawing a part of the (meth)acrylic acid-containing liquid (condensate) from the condensation column and returning it to the condensation column. The circulation line is provide in one stage of the condensation column such that the (meth)acrylic acid-containing liquid is returned from a bottom part of the stage to a top part of the stage. The circulation line may be provided, for example, in each of an upper stage, a middle stage, and a lower stage of the condensation column, or may be provided only in some of the stages. The circulation line may be provided with a heat exchanger, and the (meth)acrylic acid-containing liquid withdrawn from the condensation column may be cooled by the heat exchanger and then returned to the same stage of the condensation column. In general, no liquid medium other than the condensate generated in the condensation column is added to the condensate to be returned to the condensation column.

In the case where the easily polymerizable compound-containing liquid ((meth)acrylic acid-containing liquid in the above case) is obtained in the absorption column or the condensation column, the polymerization prevention step (or further the cleaning step) according to the present invention can be conducted in the step of introducing an easily polymerizable compound-containing gas into the cooling column selected from the absorption column and the condensation column to obtain the easily polymerizable compound-containing liquid. In this case, the gas supply means is preferably provided in the cooling column or the circulation line of the cooling column, whereby polymerization of the easily polymerizable compound in the cooling column and the circulation line thereof can be effectively suppressed, while preventing clogging of the gas supply means and accompanying decrease in the supply of oxygen-containing gas.

In the case where the gas supply means is provided as described above and the cleaning step is carried out using a liquid as the cleaning medium, it is preferable that the easily polymerizable compound-containing liquid withdrawn from the cooling column is used as that liquid and supplied to the gas supply means. Alternatively, the easily polymerizable compound-containing liquid may be withdrawn from the circulation line of the cooling column and supplied to the gas supply means.

The method for producing an easily polymerizable compound of the present invention may comprise a step of introducing an easily polymerizable compound-containing liquid into a vaporization separation column selected from a distillation column and a stripping column to purify (hereinafter referred to as a "vaporization separation step"), and the polymerization prevention step of the present invention may be conducted in this step. By introducing an easily polymerizable compound-containing liquid into a vaporization separation column and purifying it, at least a part of the components having a lower and/or higher boiling point than the easily polymerizable compound is removed from the easily polymerizable compound-containing liquid, thereby obtaining a purified easily polymerizable compound.

The easily polymerizable compound-containing liquid introduced into the vaporization separation column of the distillation column or the stripping column is not particularly limited as long as it is a liquid obtained in an easily polymerizable compound production process and contains an easily polymerizable compound. For example, in the case where the easily polymerizable compound is (meth)acrylic acid, examples of the easily polymerizable compound-containing liquid include a (meth)acrylic acid-containing liquid obtained in the collection step or the condensation step described above, a (meth)acrylic acid-containing liquid obtained in any purification step preceding the vaporization separation step, a purification residue obtained in any purification step subsequent to the vaporization separation step, and the like. Purification means that can be used in any of these purification steps include crystallization, distillation (fractional distillation), stripping, extraction and the like, which may be combined.

As the distillation column or the stripping column used as the vaporization separation column, a tower-type equipment such as a tray column in which trays (sieve trays) are provided in the column (e.g., a perforated plate column and a bubble cap column) and a packed column filled with a packing in the column is preferably used. The vaporization separation column has a top part, a middle part and a bottom part, and provided that the vaporization separation column is divided in a height direction, the range, with respect to the height direction, in which the tray or the packing is provided is referred to as the middle part, a top side thereto is referred to as the top part, and a bottom side thereto is referred to as the bottom part.

An introducing position of the easily polymerizable compound-containing liquid into the vaporization separation column and a withdrawing position of a purified easily polymerizable compound withdrawn from the vaporization separation column may be appropriately set, according to the composition of the easily polymerizable compound-containing liquid and the type of the vaporization separation column, that is, depending on whether the vaporization separation column is a distillation column or a stripping column.

In the case where the vaporization separation column is a distillation column, it is preferable that the easily polymerizable compound-containing liquid is introduced into the distillation column from the middle part of the distillation column. The withdrawing position of the purified easily polymerizable compound withdrawn from the distillation column may be appropriately set according to the composition of the easily polymerizable compound-containing liquid introduced into the distillation column.

For example, in the case where a (meth)acrylic acid-containing liquid is introduced into the distillation column and purified, it is preferable that the purified (meth)acrylic acid is withdrawn from the distillation column as follows. In the case where the distillation column is for distilling the (meth)acrylic acid-containing liquid obtained in the preceding collection step or condensation step, since the (meth)acrylic acid-containing liquid contains more amount of components with lower boiling point than (meth)acrylic acid, it is preferable to withdraw purified (meth)acrylic acid from the middle part and/or the bottom part of the distillation column. In this case, the withdrawing position of the purified (meth)acrylic acid is preferably closer to the bottom of the column than the introducing position of the (meth)acrylic acid-containing liquid. On the other hand, in the case where the (meth)acrylic acid-containing liquid contains more amount of components with higher boiling point than (meth)acrylic acid, such as Michael adducts and maleic acid, the purified (meth)acrylic acid is preferably withdrawn from a position closer to a top of the column than the introducing position of the (meth)acrylic acid-containing liquid, and more preferably withdrawn from the top part of the column. In this case, the purified (meth)acrylic acid is preferably withdrawn as vapor.

In the case where the vaporization separation column is a stripping column, it is preferable that the easily polymerizable compound-containing liquid is introduced into the stripping column from the middle part or the top part of the column and the purified easily polymerizable compound is withdrawn from the bottom part of the column. Basically, in the stripping column, a stripping gas is supplied from the bottom part of the column, low boiling point components contained in the easily polymerizable compound-containing liquid are vaporized to be separated. The purified easily polymerizable compound is preferably withdrawn from the stripping column as a liquid.

The vaporization separation column is preferably provided with a circulation line for withdrawing a part of the easily polymerizable compound-containing liquid from the vaporization separation column and returning it to the vaporization separation column. The circulation line is preferably provided so as to return a bottom liquid withdrawn from the bottom part of the column to the bottom part of the column. A return position of the circulation line to the bottom part of the column may be higher than or lower than the position at which the bottom liquid is withdrawn from the bottom part of the column, or may be at the same height as that. The circulation line is preferably provided with a reboiler, which increase temperature in the vaporization separation column and facilitates its temperature control, thereby improving separation efficiency in the vaporization separation column

In the case where the easily polymerizable compound-containing liquid is introduced into the distillation column or the stripping column to purify as described above, the polymerization prevention step (or further the cleaning step) according to the present invention can be conducted in the step of introducing the easily polymerizable compound-containing liquid into vaporization separation column selected from the distillation column and the stripping column to purify. In this case, the gas supply means is preferably provided in the vaporization separation column, a supply line for supplying the easily polymerizable compound-containing liquid to the vaporization separation column, or the circulation line of the vaporization separation column, whereby polymerization of the easily polymerizable compound in the vaporization separation column and the circulation line thereof can be effectively suppressed, while preventing clogging of the gas supply means and accompanying decrease in the supply of oxygen-containing gas. In the case where the gas supply means is provided in the circulation line of the vaporization separation column, it is preferable that the gas supply means is installed on the upstream of the reboiler in the circulation line from the viewpoint of suppressing polymerization of the easily polymerizable compound in the reboiler of the circulation line.

In the case where the gas supply means is provided as described above and the cleaning step is carried out using a liquid as the cleaning medium, it is preferable that the easily polymerizable compound-containing liquid withdrawn from the vaporization separation column is used as that liquid and supplied to the gas supply means. Alternatively, the easily polymerizable compound-containing liquid may be withdrawn from the circulation line of the vaporization separation column and supplied to the gas supply means. In the case where the gas supply means is provided in the supply line for supplying the easily polymerizable compound-containing liquid to the vaporization separation column, the easily polymerizable compound-containing liquid withdrawn from the supply line may be supplied to the gas supply means.

The method for producing an easily polymerizable compound of the present invention may comprise a step of storing an easily polymerizable compound-containing liquid in a storage tank, and the polymerization prevention step of the present invention may be conducted in this step. For example, in the case where the easily polymerizable compound-containing liquid is a (meth)acrylic acid-containing liquid, the easily polymerizable compound-containing liquid stored in the storage tank may be a (meth)acrylic acid-containing liquid obtained in the collection step or the condensation step described above, a purified (meth)acrylic acid-containing liquid or a purification residue obtained in the vaporization separation step described above, a (meth)acrylic acid-containing liquid or a purification residue obtained in any purification step preceding or subsequent to the vaporization separation step, a (meth)acrylic acid product, or the like.

In the step of storing the easily polymerizable compound-containing liquid, the gas supply means can be provided in the storage tank or a supply line for supplying the easily polymerizable compound-containing liquid to the storage tank. Thereby, polymerization of the easily polymerizable compound in the storage tank can be effectively suppressed, while preventing clogging of the gas supply means and accompanying decrease in the supply of oxygen-containing gas. In the case where the storage tank is provided with a circulation line for withdrawing a part of the easily polymerizable compound-containing liquid in the storage tank and returning it to the storage tank, the gas supply means may be provided in the circulation line.

In the case where the gas supply means is provided in the storage tank, the supply line of the storage tank, or the circulation line of the storage tank as described above, and the cleaning step is carried out using the easily polymerizable compound-containing liquid, it is preferable that the easily polymerizable-containing liquid withdrawn from the storage tank is supplied to the gas supply means. Alternatively, the easily polymerizable compound-containing liquid to be supplied to the storage tank may be supplied to the gas supply means, thereby conducting the cleaning step.

The method for producing an easily polymerizable compound of the present invention may comprise a step of introducing an easily polymerizable compound-containing liquid into a reactor and reacting it, and the polymerization prevention step of the present invention may be conducted in this step. For example, in the case where the easily polymerizable compound-containing liquid is a (meth)acrylic acid-containing liquid, the polymerization prevention step according to the present invention can be conducted in a reactor for producing a (meth)acrylic acid derivative from (meth)acrylic acid. As the (meth)acrylic acid derivative, a (meth)acrylic acid ester is typically mentioned, and examples of a (meth)acrylic acid ester include methyl (meth)acrylate, ethyl (meth)acrylate, isobutyl (meth)acrylate, t-butyl (meth)acrylate, 2-ethylhexyl (meth)acrylate, nonyl (meth)acrylate, 2-hydroxyethyl (meth)acrylate, 2-hydroxypropyl (meth)acrylate, ethylene glycol di(meth)acrylate, polyethylene glycol di(meth)acrylate, trimethylolpropane di(meth)acrylate, trimethylolpropane tri(meth)acrylate, 2-(2-vinyloxyethoxy)ethyl (meth)acrylate, methoxyethyl (meth)acrylate, benzyl (meth)acrylate, glycidyl (meth)acrylate, and the like.

In the step of introducing the easily polymerizable compound-containing liquid into the reactor and reacting it, the gas supply means can be provided in the reactor or a supply line for supplying the easily polymerizable compound-containing liquid to the reactor. Thereby, it is possible to suppress clogging of the gas supply means and the accompanying decrease in the supply amount of the oxygen-containing gas, and a desired reaction, such as esterification reaction, can be suitably carried out while suppressing polymerization reaction of the easily polymerizable compound. In the case where the reactor is provided with a circulation line for withdrawing a part of the easily polymerizable compound-containing liquid in the reactor and returning it to the reactor, the gas supply means may be provided in the circulation line.

In the case where the gas supply means is provided as described above and the cleaning step is carried out using a liquid as the cleaning medium, it is preferable that the easily polymerizable compound-containing liquid withdrawn from the reactor or the easily polymerizable compound-containing liquid to be supplied to the reactor is used as that liquid and supplied to the gas supply means.

The method for producing an easily polymerizable compound of the present invention has been described above, however, the present invention can also be applied to a method for preventing clogging of a gas supply means or a method for cleaning a gas supply means. In this case, the gas supply means has one or more supply ports as described above, and is immersed in an easily polymerizable compound-containing liquid, and an oxygen-containing gas is supplied to the easily polymerizable compound-containing liquid through the gas supply means. The gas supply means may be cleaned by supplying a gas or liquid cleaning medium to the gas supply means, thereby cleaning a cleaning port of the gas supply means.

This application claims priority to Japanese Patent Application No. 2020-098799, filed on June 5, 2020. All of the contents of the Japanese Patent Application No. 2020-098799, filed on June 5, 2020, are incorporated by reference herein.

### EXAMPLES

The present invention will be hereinafter described more specifically by reference to Examples; however, the present invention is not limited to these Examples.

(1) Experiment 1: Experiment for confirming backflow into supply pipe (1-1) Experimental method

A pipe made of SUS304 with an inner diameter of 16 mm, an outer diameter of 19 mm, and a length of 1 m was prepared, and one end of this SUS pipe was closed to make a supply pipe. A blower was connected to the other end of the SUS pipe. In Experiment No. 1-1, one circular hole (supply port) with a diameter of 1 mm was opened in the vicinity of the one end of the SUS pipe, and the SUS pipe was immersed in acrylic acid filled in a tank so that the supply port faces downward. Liquid depth from the supply port of the SUS pipe to the liquid surface was about 10 cm, and temperature of the acrylic acid was 25°C. Air was fed from the blower to the inside of the SUS pipe at 0.3 NL/min, during which the presence or absence of backflow of acrylic acid into the SUS pipe was observed with a fiberscope, and after supplying air for 10 minutes, the presence or absence of acrylic acid droplets (mist) inside the SUS pipe was checked. Experiment Nos. 1-2 to 1-13 were conducted such that air was fed to the inside of the SUS pipe for 10 minutes in the same manner as Experiment No. 1-1, except that the conditions of the supply port and air supply volume were changed as shown in Table 1.

### (1-2) Experimental results

The experimental results are shown in Table 1. In Experiment Nos. 1-2, 1-5 to 1-7, the ratio V/S of a linear velocity V (m/s) of air at the supply port to an area S (mm²) of the supply port was 1.7 to 5.5, and backflow of acrylic acid into the inside of the supply pipe (adhesion of droplets) was observed. In Experiment Nos. 1-10 to 1-11, the area S (mm²) per one supply port exceeded 20, and backflow of acrylic acid into the inside of the supply pipe (adhesion of droplets) was observed. Meanwhile, in Experiment Nos. 1-1, 1-3 to 1-4, and 1-8 to 1-9, the area S (mm²) per one supply port was 0.1 to 20, and the ratio V/S was 6.6 to 15.3, resulting in that backflow of acrylic acid into the inside of the supply pipe (adhesion of droplets) was not observed. Also in Experiment Nos. 1-12 and 1-13 with a plurality of supply ports, the same results as in Experiment Nos. 1-2 and 1-3 with single supply port were confirmed.

**[Table 1]**

| No. | | 1-1 | 1-2 | 1-3 | 1-4 | 1-5 | 1-6 | 1-7 | 1-8 | 1-9 | 1-10 | 1-11 | 1-12 | 1-13 |
|---|---|---|---|---|---|---|---|---|---|---|---|---|---|---|
| diameter of supply port | mm | 1.0 | 2.0 | 2.0 | 2.0 | 3.0 | 3.0 | 3.0 | 3.0 | 4.0 | 7.0 | 7.0 | 2.0 | 2.0 |
| number of supply port | - | 1 | 1 | 1 | 1 | 1 | 1 | 1 | 1 | 1 | 1 | 1 | 2 | 2 |
| area per one supply port [S] | mm² | 0.8 | 3.1 | 3.1 | 3.1 | 7.1 | 7.1 | 7.1 | 7.1 | 12.6 | 38.5 | 38.5 | 3.1 | 3.1 |
| air supply volume | NL/min | 0.3 | 1.1 | 2.5 | 3.7 | 2.5 | 5.7 | 8.3 | 10.0 | 32.7 | 100 | 300 | 2.3 | 5.1 |
| air linear velocity at supply port [V] | m/s | 12 | 12 | 27 | 39 | 12 | 27 | 39 | 47 | 87 | 87 | 260 | 12 | 27 |
| ratio V/S | (m/s) /mm² | 15.3 | 3.8 | 8.6 | 12.4 | 1.7 | 3.8 | 5.5 | 6.6 | 6.9 | 2.3 | 6.8 | 3.8 | 8.6 |
| backflow (mist) observed | - | No | Yes | No | No | Yes | Yes | Yes | No | No | Yes | Yes | Yes | No |

### (2) Experiment 2: Experiment for eliminating clogging of supply pipe by air blow (2-1) Experimental method

A pipe made of SUS304 with an inner diameter of 16 mm, an outer diameter of 19 mm, and a length of 1 m was prepared, and one end of this SUS pipe was closed to make a supply pipe. In Experiment No. 2-1, one circular hole (supply port) with a diameter of 1 mm was opened in the vicinity of the one end of the SUS pipe, and this SUS pipe was installed, as an air supply pipe for preventing polymerization of acrylic acid, so that the supply port faces downward, in a circulation line of a low-boiling distillation column in an acrylic acid production facility for one month, resulting in that the supply port was clogged. A compressor was connected to the other unclosed end of the SUS pipe whose supply port was clogged, and air was fed to the inside of the SUS pipe at 52 NL/min for 0.1 second to confirm whether or not the clogging was eliminated. Experiment Nos. 2-2 to 2-6 were conducted in the same manner as Experiment No. 2-1, except that the conditions of the supply port and air supply volume were changed as shown in Table 2, and air was fed to the inside of the SUS pipe after clogging of the supply port to confirm whether or not the clogging was eliminated.

### (2-2) Experimental results

The results are shown in Table 2. In Table 2, in the case where the clogging was eliminated, the column of "clogging eliminated" is marked with "O", and in the case where the clogging was not eliminated, it is marked with "X". Regardless of the size of the supply port provided in the supply pipe, clogging of the supply port in the supply pipe could be eliminated by supplying air to the supply pipe so that the pressure difference at the supply port was 200 kPa or more.

**[Table 2]**

| No. | | 2-1 | 2-2 | 2-3 | 2-4 | 2-5 | 2-6 |
|---|---|---|---|---|---|---|---|
| diameter of supply port | mm | 1.0 | 2.0 | 2.0 | 3.0 | 3.0 | 4.0 |
| area of supply port | mm² | 0.8 | 3.1 | 3.1 | 7.1 | 7.1 | 12.6 |
| air supply volume | NL/min | 52 | 37 | 89 | 116 | 201 | 207 |
| air linear velocity at supply port | m/s | 548 | 387 | 949 | 548 | 949 | 548 |
| pressure difference across supply port | kPa | 200 | 100 | 600 | 200 | 600 | 200 |
| elimination of clogging | - | ○ | X | ○ | ○ | ○ | ○ |

## Claims

1. A method for producing an easily polymerizable compound, comprising a polymerization prevention step of supplying an oxygen-containing gas to an easily polymerizable compound-containing liquid through a gas supply means having one or more supply ports, wherein
an area S (mm²) per one supply port is 0.1 or more and 20 or less, and
a ratio V/S of a linear velocity V (m/s) of the gas at the supply port to the area S in the polymerization preventing step is 6.0 or more.

2. The method for producing an easily polymerizable compound according to claim 1, further comprising a cleaning step of supplying a gas to the gas supply means to clean the supply port, wherein
a pressure difference across the supply port of the gas supply means in the polymerization prevention step is 100 kPa or lower, and
a pressure difference across the supply port of the gas supply means in the cleaning step is 150 kPa or higher.

3. The method for producing an easily polymerizable compound according to claim 1 or 2, further comprising a cleaning step of supplying a liquid to the gas supply means to clean the supply port.

4. A method for producing an easily polymerizable compound, comprising a polymerization prevention step of supplying an oxygen-containing gas to an easily polymerizable compound-containing liquid through a gas supply means having one or more supply ports, and a cleaning step of supplying a gas to the gas supply means to clean the supply port, wherein
a pressure difference across the supply port of the gas supply means in the polymerization prevention step is 100 kPa or lower, and
a pressure difference across the supply port of the gas supply means in the cleaning step is 150 kPa or higher.

5. A method for producing an easily polymerizable compound, comprising a polymerization prevention step of supplying an oxygen-containing gas to an easily polymerizable compound-containing liquid through a gas supply means having one or more supply ports, and a cleaning step of supplying a liquid to the gas supply means to clean the supply port.

6. The method for producing an easily polymerizable compound according to any one of claims 1 to 5, wherein the polymerization prevention step is conducted in at least one of the following manners (1) to (4):
(1) the polymerization prevention step is conducted in a step of introducing an easily polymerizable compound-containing gas into a cooling column selected from an absorption column and a condensation column to obtain the easily polymerizable compound-containing liquid, and
the gas supply means is provided in the cooling column or a circulation line for withdrawing a part of the easily polymerizable compound-containing liquid from the cooling column and returning it to the cooling column;
(2) the polymerization prevention step is conducted in a step of introducing the easily polymerizable compound-containing liquid to a vaporization separation column selected from a distillation column and a stripping column to purify, and
the gas supply means is provided in the vaporization separation column, a supply line for supplying the easily polymerizable compound-containing liquid to the vaporization separation column, or a circulation line for withdrawing a part of the easily polymerizable compound-containing liquid from the vaporization separation column and returning it to the vaporization separation column;
(3) the polymerization prevention step is conducted in a step of storing the easily polymerizable compound-containing liquid in a storage tank, and
the gas supply means is provided in the storage tank, a supply line for supplying the easily polymerizable compound-containing liquid to the storage tank, or a circulation line for withdrawing a part of the easily polymerizable compound-containing liquid from the storage tank and returning it to the storage tank;
(4) the polymerization prevention step is conducted in a step of introducing the easily polymerizable compound-containing liquid in a reactor and reacting it, and
the gas supply means is provided in the reactor, a supply line for supplying the easily polymerizable compound-containing liquid to the reactor, or a circulation line for withdrawing a part of the easily polymerizable compound-containing liquid from the reactor and returning it to the reactor.
